# EUROPEAN PATENT SPECIFICATION

(11) **EP 2 170 708 B1**
(45) Date of publication and mention of the grant of the patent: **24.10.2012**
(21) Application number: 08770629.7
(22) Date of filing: 11.06.2008
(51) Int. Cl.: A61K 9/00, C11D 1/00, B65B 25/00, A61L 12/08, A45C 11/00, C11D 1/62, C11D 3/37, C11D 3/00

(54) **OPHTHALMIC SOLUTIONS**
OPHTHALMISCHE LÖSUNGEN
SOLUTIONS OPHTALMIQUES

(30) Priority: 22.06.2007 US 767045
(43) Date of publication of application: 07.04.2010
(73) Proprietor: Bausch & Lomb Incorporated, Rochester, NY 14604-2701 (US)
(72) Inventor: LAI, Yu-Chin, Pittsford, NY 14534 (US); LANG, Weihong, Amston, CT 06231 (US)
(74) Representative: Glas, Holger
(86) International application number: PCT/US2008/066470
(87) International publication number: WO 2009/002703

(56) References cited:
- WO-A-97/43373
- WO-A-99/29818
- WO-A-2006/038080
- WO-A-2007/070653

## Description

The present invention generally relates to ophthalmic solutions such as packaging solutions and multi-purpose solutions.

### 2. Description of the Related Art

Copolymers derived from a monomer bearing at least a center of permanent positive charge and an ethylenically unsaturated monomer are known. For example, U.S. Patent Nos. 5,648,442; 5,705,583 and 5,783,650 disclose a polymer obtainable by copolymerizing an ethylenically unsaturated, comonomer containing a group bearing a centre of permanent positive charge, which can either be cationic or, more preferably, zwitterionic; and an ethylenically unsaturated, comonomer bearing an ionic group capable of binding to a surface by ionic interaction. These patents further disclose that the polymer may also be copolymerized with a diluent monomer such as N-vinylpyrrolidone (NVP), hydroxyl or polyhydroxyl (alk)acrylate, e.g., sorbitol methacrylate. These patents also disclose that the polymer products disclosed therein are useful for coating a surface such as a finished implant, prosthesis, membrane, catheter, contact lens, intraocular lens, to impart biocompatibility. A contact lens cleaning composition a zwitterionic copolymer is known from WO 99/29818.

U.S. Patent Application Publication No. 2002/0165324 ("the '324 application") discloses a crosslinked copolymer for use as a contact lens which is obtainable by polymerizing a neutral diluent monomer or monomers, a monomer or monomers bearing a centre of permanent positive charge and a bifunctional or trifunctional crosslinking agent. The '324 application further discloses the diluent monomer can be NVP or a hydroxyalkyl (alk)acrylate, e.g., 2-hydroxyethyl(alk)acrylate and the monomer or monomers bearing a centre of permanent positive charge can be cationic or zwitterionic.

Blister-packs and glass vials are used to individually package each soft contact lens for sale to the customer. Saline or deionized water is commonly used to store the lens in the blister-packs, as mentioned in various patents related to the packaging or manufacturing of contact lenses. Because lens material may tend to stick to itself and to the lens package, packaging solutions for blister-packs have sometimes been formulated to reduce or eliminate lens folding and sticking. For this reason, polyvinyl alcohol (PVA) has been used in contact-lens packaging solutions.

It has been stated that if a lens is thoroughly cleaned before insertion, lacrimal fluid can adequately wet the lens. Furthermore, the difficulties of adding a surfactant to a packaging solution, including the possibility of lowering shelf-life and/or adverse reactions during heat sterilization, have further limited the use of surfactants in a packaging solution for the purpose of providing any possible or marginal effect on lens comfort. It is only after a lens has been worn, when proteins or other deposits have formed on the surface of the lens, that surfactants have been used in standard lens-care solutions.

It is highly desirable that contact lenses be as comfortable as possible for wearers. Manufacturers of contact lenses are continually working to improve the comfort of the lenses. Nevertheless, many people who wear contact lenses still experience dryness or eye irritation throughout the day and particularly towards the end of the day. An insufficiently wetted lens at any point in time will cause significant discomfort to the lens wearer. Although wetting drops can be used as needed to alleviate such discomfort, it would certainly be desirable if such discomfort did not arise in the first place.

Accordingly, it would be desirable to provide an improved packaging system for storing ophthalmic devices such as an ophthalmic lens that would be comfortable to wear in first actual use and allow for extended wear of the lens without irritation or other adverse effects to the cornea. It would further be desirable to provide improved lens care solutions that can be distilled directly in the eye such as for rewetting a contact lens while worn or instilled indirectly in the eye, such as contact lens treating solutions for treating the contact lens prior to the lens being inserted on the eye.

### SUMMARY OF THE INVENTION

In accordance with one embodiment of the present invention, a packaging system for the storage of an ophthalmic device as defined in claim 1 is provided comprising a sealed container containing one or more unused ophthalmic devices immersed in an aqueous packaging solution comprising a polymerization product obtained from a monomeric mixture comprising (a) a monomer bearing at least a center of permanent positive charge and (b) a non-ionic ethylenically unsaturated monomer, wherein the solution has an osmolality of at least about 200 mOsm/kg, a pH of about 4 to about 9 and is heat sterilized.

In accordance with another embodiment of the present invention as defined in claim 12, a method of treating a biomedical device is provided, the method comprising:
contacting a biomedical device with a solution comprising a polymerization product obtained from a monomeric mixture comprising (a) a monomer bearing a center of permanent positive charge and (b) a non-ionic ethylenically unsaturated monomer.

In accordance with an additional embodiment of the present invention as defined in claim 16, a multi-purpose ophthalmically acceptable solution is provided comprising (a) a copolymer comprising one or more first monomeric segments having a cationic charge and one or more second monomeric non-ionic segments; and (b) an ophthalmically acceptable carrier for the copolymer.

### DETAILED DESCRIPTION OF THE PREFERRED EMBODIMENTS

The present invention is directed to ophthalmic solutions intended for application in the eye or intended for treating a biomedical device to be placed in contact with the eye such as a contact lens. Ophthalmic solutions specifically include compositions for direct instillation in the eye, including eye drop solutions such as for treating dry eye, and contact lens treating solutions distilled directly in the eye such as for rewetting a contact lens while worn as well as those that also qualify as a multi-purpose solution. Ophthalmic compositions also include compositions instilled indirectly in the eye, such as contact lens treating solutions for treating the contact lens prior to the lens being inserted on the eye or a packaging solution for storing the lens.

The ophthalmically acceptable solutions according to the present invention are physiologically compatible. Specifically, the compositions must be "ophthalmically safe" for use with a contact lens, meaning that a contact lens treated with the solution is generally suitable and safe for direct placement on the eye without rinsing, that is, the solution is safe and comfortable for daily contact with the eye via a contact lens that has been wetted with the solution. An ophthalmically safe composition has a tonicity and pH that is compatible with the eye and comprises materials, and amounts thereof, that are non-cytotoxic according to ISO (International Standards Organization) standards and U.S. FDA regulations. The compositions should be sterile in that the absence of microbial contaminants in the product prior to release must be statistically demonstrated to the degree necessary for such products.

In general, the ophthalmic solutions of the present invention will include at least a polymerization product obtained from a monomer mixture comprising (a) a monomer bearing at least a center of permanent positive charge and (b) a non-ionic ethylenically unsaturated monomer. The comonomer bearing the centre of permanent positive charge is a zwitterionic monomer. The monomer includes within its structure not only a centre of permanent positive charge but also a centre of negative charge.

Representative examples of a monomer which bears at least a centre of positive charge are of general Formula I:

Y-B-X (I)

wherein B is a bond, a straight or branched, substituted or unsubstituted alkylene, substituted or unsubstituted oxaalkylene, or substituted or unsubstituted oligooxaalkylene chain; X is a group bearing a centre of permanent positive charge and Y is an ethylenically unsaturated polymerizable group.

In general, group X bearing a centre of permanent positive charge can be of the general formula: -Z¹-N⁺(R¹)₃, -Z¹-P⁺(R²)₃, -Z¹-S⁺(R²)₂, or -Z¹-Het⁺, wherein Z¹ is a substituted or unsubstituted alkylene group of 1 to about 12 carbon atoms, substituted or unsubstituted disubstituted-arylene group, substituted or unsubstituted alkylene arylene group, substituted or unsubstituted arylene alkylene group, substituted or unsubstituted alkylene aryl alkylene group, substituted or unsubstituted cycloalkylene group, substituted or unsubstituted alkylene cycloalkyl group, substituted or unsubstituted cycloalkyl alkylene group or substituted or unsubstituted alkylene cycloalkyl alkylene group; R¹ independently is hydrogen or a substituted or unsubstituted alkyl group of 1 to 4 carbon atoms, preferably methyl, or substituted or unsubstituted aryl, such as substituted or unsubstituted phenyl, or two of the R¹ groups together with the nitrogen atom to which they are attached form an aliphatic heterocyclic ring containing from 5 to 7 atoms, or the three R¹ groups together with the nitrogen atom to which they are attached form a fused ring structure containing from 5 to 7 atoms in each ring; R² independently is R¹ or a group OR', wherein R¹ is as defined above; and Het is an aromatic nitrogen-, phosphorus- or sulphur-containing C₃-C₃₀ ring such as, for example, pyridine.

X is a zwitterionic group, and the centre of permanent positive charge can include a group X which is of the general Formulae IIA-IIE, in which the group of Formula IIA is as follows: 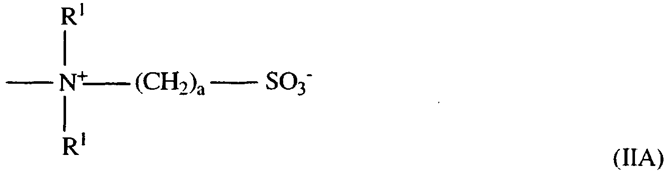 wherein a is 1 to 4 and R¹ has the aforestated meanings;
the group of Formula IIB is as follows: 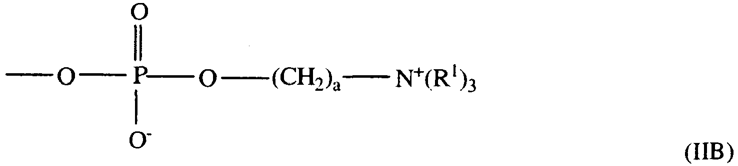 wherein a and R¹ has the aforestated meanings;
the group of Formula IIC is as follows: 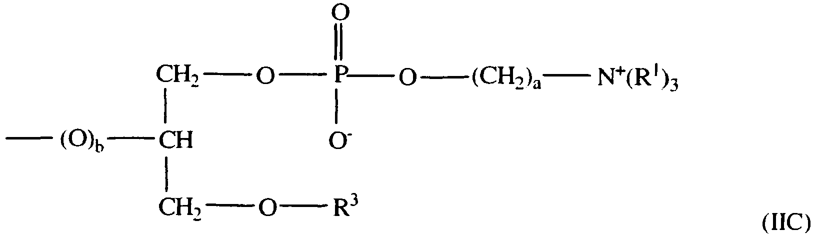 wherein a and R¹ have the aforestated meanings, R³ is hydrogen, a group of the general formula -C(O)B¹R⁴ wherein R⁴ is hydrogen or methyl, B¹ is a valence bond or straight or branched alkylene, oxaalkylene or oligo-oxaalkylene group, or a group of the general formula -[C(O)]_{c}(CR⁵)_{d}(SiR⁶₂)(OSi R⁶₂)ₑR⁶ in which each group R⁵ is the same or different and is hydrogen or alkyl of 1 to 4 carbon atoms, each group R⁶ is the same or different and is alkyl of 1 to 4 carbon atoms or C₅-C₃₀ aralkyl such as benzyl or phenylethyl, c is 0 or 1, d is from 0 to 6 and preferably 2 to 4 with the proviso that c and d are not both 0, and e is from 0 to 49 and preferably 4 to 29; and if B is other than a valence bond b is 1 and if B is a valence bond b is 0, if X is directly bonded to an oxygen or nitrogen atom and otherwise b is 1;
the group of Formula IID is as follows: 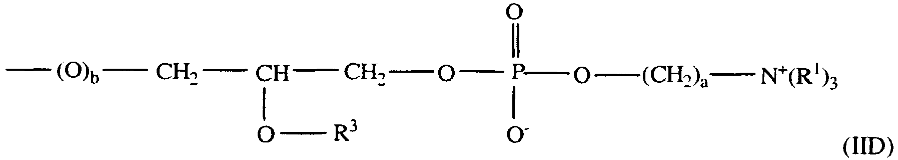 wherein a, R¹ and R³ have the aforestated meanings; and
the group of Formula IIE is as follows: 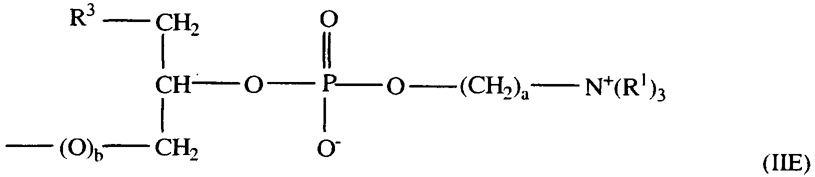 wherein a, R¹ and R³ have the aforestated meanings.

Representative examples of group B of Formula I include:
an alkylene group of the formula -(CR⁷₂)_{f}-, wherein each group -(CR⁷₂)- is the same or different, and wherein R⁷ in each group is the same or different and is hydrogen, fluorine or a substituted or unsubstituted C₁-C₄ alkyl or fluoroalkyl group with hydrogen being preferred, and f is from 1 to 12, and preferably 1 to 6;
an oxaalkylene group such as alkoxyalkyl having 1 to 6 carbon atoms in each alkyl moiety, e.g., -CH₂O(CH₂)₄-; or
an oligo-oxaalkylene group of the formula -[(CR⁸₂)_{g}O]ₕ(CR⁸₂)_{c}- wherein each group -(CR⁸₂)- is the same or different and wherein R⁸ in each group is the same or different and is hydrogen, fluorine or a substituted or unsubstituted C₁-C₄ alkyl or fluoroalkyl group with hydrogen being preferred, and g is from 1 to 6, and preferably 2 or 3 and h is from 2 to 11, and preferably 2 to 5. Preferred group B includes alkylene, oxaalkylene and oligo-oxaalkylene groups of up to 24 carbon atoms optionally containing one or more fluorine atoms. In one embodiment, group B can contain from 6 to 24 carbon atoms and preferably from 6 to 18 carbon atoms.

Representative examples of group Y of Formula I include ethylenically unsaturated polymerizable groups of the general formulae: 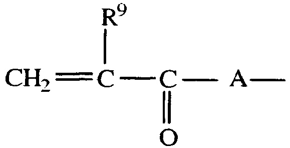 and 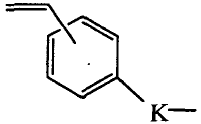 wherein R⁹ is hydrogen or a substituted or unsubstituted C₁-C₄ alkyl group or fluoroalkyl group; A is -O- or -NR¹⁰- wherein R¹⁰ is hydrogen or a substituted or unsubstituted C₁-C₄ alkyl group or R¹⁰ is -B-X where B and X are as defined above. K can be a group of the formulae -(CH₂)ᵢCO(O)-, -(CH₂)ᵢC(O)O-, -(CH₂)ᵢCOC(O)O-, -(CH₂)ᵢC NR¹¹-, -(CH₂)ᵢNR¹¹C(O)-, -(CH₂)ᵢC(O)NR¹¹-, (CH₂)ᵢNR¹¹C(O)O-, -(CH₂)ᵢOC(O)NR¹¹-, -(CH₂)ᵢNR¹¹C(O)NR¹¹, -(CH₂)ᵢO-, -(CH₂)ᵢSO₃-, or, optionally in a combination with B, a valence bond, and i is from 1 to 12 and R¹¹ is the same or different and is hydrogen or a substituted or unsubstituted C₁-C₄ alkyl group.

The proviso on whether B may be a valence bond ensures that the centre of permanent positive charge in X is not directly bonded to a heteroatom, such as an oxygen or nitrogen atom in Y.

Preferred monomers which bear a centre of positive charge are those of general Formula III or IV: 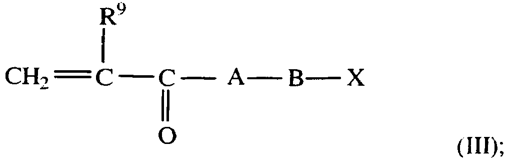 or 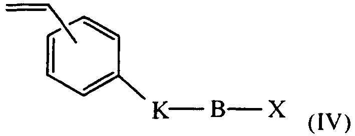 wherein R⁹, A, K, B and X have the aforestated meanings.

Preferred zwitterionic groups for instance are groups on ethylenically unsaturated monomer YBX in which the cationic moiety is based on a quaternary ammonium group and the anionic moiety is based on a phosphate group, e.g., ammonium phosphate ester zwitterionic groups. Typically, the cationic group is located at the end of pendant group X distant from B. Most preferred zwitterionic groups are of Formulae IIA, IIB, IIC, IID and IIE as defined above. Of these groups, the group of Formula IIB is particularly preferred.

Monomers containing a zwitterionic group such as those of Formulae II and III may be prepared by conventional techniques using known reactions. See, for example, U.S. Patent No. 5,712,326 for processes for preparing zwitterionic monomers. For example, compounds of Formulae II and III can be prepared using a suitable substituted alkyl (alk)acrylate or suitable substituted styrene as a precursor. Examples of suitable substituted alkyl (alk)acrylates include dimethylaminoethyl(meth)acrylate and 2-hydroxyethyl(meth)acrylate.

Monomers of Formulae II and III containing a group of Formula IIC in which R³ is - C(O)B¹R⁴ may be prepared by selective acylation of glycerophosphorylcholine or analogues thereof at the primary hydroxyl group with an activated acid derivative such as an acid anhydride O(C(O)B¹R⁴)₂ or an acid halide R⁴B¹COHal where B¹ and R⁴ are as defined above and Hal is halogen, followed by acylation of the secondary hydroxyl group with an appropriate acylating agent, for example, methacryloyl chloride. If desired, purification, e.g., by column chromatography on a suitable support, may be performed after each acylation or after the second acylation only. Suitable activated acid derivatives include acid anhydrides, acid halides, reactive esters and imidazolides. The acylations may be performed in a suitable anhydrous, aprotic solvent, for example N,N-dimethylformamide, optionally in the presence of a suitable non-nucleophilic base, for example, triethylamine.

Alternatively, the primary alcohol group in glycerophosphoryl choline or an analogue thereof may be blocked by reaction with a suitable protecting group reagent, for example, t-butyldimethylsilyl chloride, under standard conditions and the secondary hydroxy group then treated with an acylating agent such as methacryloyl chloride. The t-butyldimethylsilyl protecting group may be removed by treatment with a dilute organic or mineral acid, for example, g-toluene sulphonic acid, hydrochloric acid or with tetrabutylammonium fluoride. The deblocked primary hydroxyl group may then be treated with an activated acid derivative such as an acid anhydride O(C(O)B¹R⁴)₂ or acid halide R⁴B¹COHal where B¹ and R⁴ are as defined above, and Hal is halogen.

Analogues of glycerophosphorylcholine (compounds of Formulae II or III containing a group of Formula IID where R³ is hydrogen) may be prepared by reaction of phosphorus oxychloride with a bromoalcohol in an inert aprotic solvent, such as dichloromethane, to give a bromoalkylphosphorodichloridate. The dichloro derivative thus produced may then be treated with an appropriately protected glycerol derivative, for example, 2,2-dimethyl 1,3-dioxolane-4-methanol, in the presence of a base, for example, triethylamine, followed by acid hydrolysis to give a bromoalkylphosphoro-glycerol derivative. This may then be treated with an amine NR¹₃, wherein R¹ is as defined above, for example, trimethylamine, to generate the glycerophosphorylcholine analogue.

Monomers of Formulae II or III containing a group of Formula IID in which R³ is - C(O)B¹R⁴ may be prepared by the selective acylation of glycerophosphorylcholine or an analogue thereof at the primary hydroxyl group with, for example, methacryloyl chloride, followed by reaction at the secondary hydroxyl group using an activated acid derivative, such as an acid halide an acid anhydride O(C(O)B¹R⁴)₂ or acid halide R⁴B¹COHal where B¹ and R⁴ are as defined above and Hal is halogen. The intermediates and final products may be purified, as necessary, using, for example, column chromatography. Optionally, protecting group strategy, similar to that outlined above in relation to production of monomers containing a group of formula IIC may be employed.

Monomers of Formulae II or III containing a group of Formula IIE may be prepared in an analogous manner to monomers containing groups of Formulae IIC or IID.

The non-ionic ethylenically unsaturated monomer is as defined in the independent claims.

Examples of non-ionic ethylenically unsaturated monomer include, but are not limited to, a hydroxyalkyl (alk)acrylate or acrylamide such as hydroxyalkyl (alk)acrylates containing from 1 to 4 carbon atoms in the hydroxyalkyl moiety, e.g., a 2-hydroxyethyl (alk)acrylate; a N-vinyl lactam, containing from 5 to 7 atoms in the lactam ring, e.g., a vinyl pyrrolidone such as N-vinyl pyrrolidone; a polyhydroxyl (alk)acrylates having 2 to 10 hydroxyl groups and preferably 2 to 6 hydroxyl groups and the alkyl group contains from 1 to 6 carbon atoms, e.g., glycerol -acrylates, -methacrylates, - ethacrylates, -acrylamides, -methacrylamides and -ethacrylamides, and the like and mixtures thereof.

The copolymers disclosed herein can be obtained by copolymerizing the monomer mixture containing at least (a) one or more of the foregoing zwitterionic monomers bearing at least a center of permanent positive charge and (b) one or more of the foregoing non-ionic ethylenically unsaturated monomers by conventional techniques for polymerization, typically thermal or photochemical polymerization, or are commercially available from such sources as Aldrich Chemical Company (Milwaukee. Wisonsin) such as a copolymer of NVP and dimethylaminoethyl methacrylate, quatemized with diethyl sulfate. Alternatively, copolymers for use herein bearing a permanent charge ( zwitterionic copolymer) can be prepared by copolymerizing a monomer capable of bearing at least a center of permanent positive charge, e.g., a tertiary amine-containing monomer, and a non-ionic ethylenically unsaturated monomer followed by quaternization of the amine portion of the copolymer to form a quaternary ammonium salt in the case where the monomer capable of bearing at least a center of permanent positive charge is a tertiary amine-containing monomer. Thus, as one skilled in the art will readily appreciate, a monomer which ultimately provides cationic character in the final copolymer can be a cationic monomer before copolymerization, or can be a monomer which is non-ionic, but becomes cationic after quaternization of the non-ionic copolymer. For example, a copolymer derived from a tertiary amine-containing monomer and non-ionic ethylenically unsaturated comonomer other than an amine, can be quatemized to form a copolymer which is the same as the copolymerization product of an ammonium salt-containing monomer and non-ionic ethylenically unsaturated comonomer. Accordingly, in one embodiment, a copolymer for use in the ophthalmic solutions according to the present invention can comprise one or more first monomeric segments having a cationic charge and one or more second monomeric non-ionic segments.

For thermal polymerization a temperature from about 40°C to about 100°C, and typically about 50°C to about 80°C is used. For photochemical polymerization, radiation such as gamma, U.V., visible, or microwave radiation may be used. Typically U.V. radiation of wavelength about 200 to about 400 nm is used.

The polymerization is generally performed in a reaction medium, which is for instance a solution or dispersion using as a solvent, for example, water or an alkanol containing from 1 to 4 carbon atoms such as methanol, ethanol or propan-2-ol. Alternatively, a mixture of any of the above solvents may be used.

The polymerization may be carried out in the presence of one or more polymerization initiators, usually free radical generators, usually peroxides or azo initiators, such as benzoyl peroxide, 2,2'-azo-bis(2-methylpropionitrile) or benzoin methyl ether. Other polymerization initiators which may be used are disclosed in, for example, "Polymer Handbook", 3rd edition, Ed. J. Brandrup and E. H. Immergut, Pub. Wiley-Interscience, New York, 1989.

Generally, polymerization can be carried out for about 1 to about 72 hours and preferably about 8 to about 48, and under an inert atmosphere of, for example, nitrogen or argon. If desired, the resulting polymer can be dried under vacuum, e.g., for about 5 to about 72 hours or left in an aqueous solution prior to use. The resulting polymerization product can have a number average molecular weight from about 5,000 to about 1,500,000 and preferably from about 15,000 to about 400,000

The precise proportion and nature of the various comonomers used in the monomeric mixture to prepare a copolymer disclosed herein may be adjusted to provide a copolymer which is particularly suitable for use in an ophthalmic solution according to the present invention. The monomeric mixture which is subjected to polymerization to provide a polymerization product according to the invention can contain a minimum of about 1 %, preferably about 10 %, more preferably about 25% by weight of monomer or monomers bearing a center of permanent positive charge and a maximum of about 99%, preferably about 90%, more preferably about 75% by weight of other non-ionic ethylenically unsaturated monomer.

In general, the copolymers disclosed herein can be present in the ophthalmic solutions of the present invention in an amount ranging from about 0.005 % to about 1% w/w and preferably from about 0.01 to about 0.5% w/w. A packaging system for the storage of an ophthalmic device ordinarily includes at least a sealed container containing one or more unused ophthalmic devices immersed in an aqueous packaging solution in accordance with the present invention. Preferably, the sealed container is a hermetically sealed blister-pack, in which a concave well containing a contact lens is covered by a metal or plastic sheet adapted for peeling in order to open the blister-pack. The sealed container may be any suitable generally inert packaging material providing a reasonable degree of protection to the lens, preferably a plastic material such as polyalkylene, PVC, polyamide, and the like.

As previously stated, aqueous packaging solutions are physiologically compatible. The pH of the aqueous packaging solutions should be maintained within the range of about 6.0 to about 9, and preferably about 6.5 to about 7.8. Suitable buffers may be added, such as boric acid, sodium borate, potassium citrate, citric acid, sodium bicarbonate, tris(hydroxymethyl)aminomethane, and various mixed phosphate buffers, e.g., combinations of Na₂HPO₄, NaH₂PO₄ and KH₂PO₄, and the like and mixtures thereof. Generally, buffers will be used in amounts ranging from about 0.05 to about 2.5 % by weight, and preferably from about 0.1 to about 1.5 % by weight of the solution.

Typically, the aqueous packaging solutions are also adjusted with tonicity agents, to approximate the osmotic pressure of normal lacrimal fluids. The solutions are made substantially isotonic with physiological saline used alone or in combination, otherwise if simply blended with sterile water and made hypotonic or made hypertonic the lenses will lose their desirable optical parameters. Correspondingly, excess saline may result in the formation of a hypertonic solution which will cause stinging and eye irritation.

Examples of suitable tonicity adjusting agents include, but are not limited to, sodium and potassium chloride, dextrose, glycerin, calcium and magnesium chloride and the like and mixtures thereof. These agents are typically used individually in amounts ranging from about 0.01 to about 2.5 % w/v and preferably from about 0.2 to about 1.5 % w/v. Generally, a 0.9 % solution of sodium chloride is equivalent in osmolality to a 3 percent of glycerol solution or a 5 percent solution of monosaccharide, so the amount of a specific agent will vary depending on the agent used. Preferably, the tonicity agent will be employed in an amount to provide a final osmotic value of at least about 200 mOsm/kg, preferably from about 200 to about 400 mOsm/kg, and more preferably from about 250 to about 350 mOsm/kg.

By the term "disinfecting agent" herein is meant a microbicidal compound that is effective for reducing or substantially eliminating the presence of an array of microorganisms present in a contact lens, which can be tested by challenging a contact lens with a specified innoculum of such microorganism. An effective amount of disinfecting agent is an amount which will at least partially reduce the microorganism population in the formulations employed, specifically a disinfecting amount is that which will reduce the microbial burden by two log orders in four hours and more preferably by one log order in one hour (without rubbing), in accordance with the FDA Chemical Disinfection Efficacy Test-July, 1985 Contact Lens Solution Draft Guidelines. In the preferred embodiment of a packing solution , the solution is heat sterilized and packaged for sale in the absence of an effective amount of disinfecting agent.

If desired, one or more additional components can be included in the aqueous packaging solutions. Such additional component or components are chosen to impart or provide at least one beneficial or desired property to the packaging solution. Such additional components may be selected from components which are conventionally used in one or more ophthalmic device care compositions. Examples of such additional components include comfort agents, surfactants, cleaning agents, wetting agents, nutrient agents, sequestering agents, viscosity builders, contact lens conditioning agents, antioxidants, and the like and mixtures thereof. These additional components may each be included in the packaging solutions in an amount effective to impart or provide the beneficial or desired property to the packaging solutions. For example, such additional components may be included in the packaging solutions in amounts similar to the amounts of such components used in other, e.g., conventional, contact lens care products.

The surfactant should be soluble in the lens care solution, not become turbid, and should be non-irritating to eye tissues. In one embodiment, the surfactant comprises at least about 90 weight percent of poly(oxyethylene) and poly(oxypropylene) segments, preferably at least 95 to 100 weight percent of poly(oxyethylene) and poly(oxypropylene) segments in one or more block copolymer chains, wherein the weight average molecular weight of said surfactant is from about 4000 to about 30,000 and wherein at least about 40 percent of said segments are poly(oxyethylene) segments. A preferred surfactant, for use as a comfort agent in contact-lens packing solutions, comprises a plurality of poly(oxyalkylene) chains, each of the poly(oxyalkylene) chains comprising a block copolymer of poly(oxyethylene) and poly(oxypropylene) segments, wherein the weight average molecular weight of said surfactant is from about 7500 to about 25,000 and wherein at least about 40 percent of said chains are poly(oxyethylene). Preferably, the number of chains is 2 to 6 and may be attached to a central moiety containing one or more, preferably 1-3, nitrogen atoms. One non-ionic surfactant that has been found to be particularly advantageous consists of a poly(oxypropylene)-poly(oxyethylene) adduct of ethylene diamine and has a molecular weight from about 7,500 to about 25,000 wherein at least 40 weight percent of said adduct is poly(oxyethylene). The CTFA Cosmetic Ingredient Dictionary's adopted name for this group of surfactants is poloxamine. Such surfactants are available from BASF Wyandotte Corp., Wyandotte, Mich., under the registered trademark "Tetronic". Examples of suitable poloxamers are Pluronic^{®} F108, F88, F68, F68LF, F127, F87, F77, P85, P75, P104, and P84. Examples of suitable poloxamines are Tetronic^{®} 707, 1107 and 1307. The comfort agent can be employed in amounts ranging from about 0.005 to about 5.0 percent, and preferably about 0.01 to about 1.0 percent by weight of the composition or solution.

Optionally, other non-ionic surfactants may be included in the packing solution in combination with the above-described comfort agents, for example, polyethylene glycol esters of fatty acids, e.g. coconut, polysorbate, polyoxyethylene or polyoxypropylene ethers of higher alkanes (C₁₂-C₁₈). Examples include Tween^{®} 20 (polysorbate 20) and Tween^{®} 80, polyoxyethylene (23) lauryl ether (Brij^{®} 35), polyoxyethyene (40) stearate (Myrj^{®} 52), polyoxyethylene (25) propylene glycol stearate (Atlas^{®} G 2612).

If desired, an amphoteric, cationic, or anionic surfactant may also be present in combination with the present comfort agent: Amphoteric surfactants suitable for use in a composition according to the present invention include materials of the type are offered commercially under the trade name "Miranol". Another useful class of amphoteric surfactants may be exemplified by the following chemical structure are exemplified by cocoamidopropyl betaine commercially available under the trade name Amphoso CA.

Surfactants suitable for use in the invention can be readily ascertained, in view of the foregoing description, from McCutcheon's Detergents and Emulsifiers, North American Edition, McCutcheon Division, MC Publishing Co., Glen Rock, N.J. 07452 and the CTFA International Cosmetic Ingredient Handbook, Published by The Cosmetic, Toiletry, and Fragrance Association, Washington, D.C.

Useful sequestering agents include, but are not limited to, disodium ethylenediaminetetraacetic acid (EDTA), alkali metal hexametaphosphate, citric acid, sodium citrate and the like and mixtures thereof.

Useful viscosity builders include, but are not limited to, hydroxyethylcellulose, methylcellulose, hydroxypropylmethylcellulose, poly(N-vinylpyrrolidone), guar, hydroxyethylguar, hydroxypropylguar, poly(vinyl alcohol) and the like and mixtures thereof. Such viscosity builders may be used in an amount of from about 0.01 to about 4.0 weight percent or less.

Useful antioxidants include, but are not limited to, sodium metabisulfite, sodium thiosulfate, N-acetylcysteine, butylated hydroxyanisole, butylated hydroxytoluene and the like and mixtures thereof.

A system for the storage and delivery of a contact lens according to the present invention comprises a sealed container containing one or more unused contact lens immersed in the above-described aqueous packaging solution. Preferably, the sealed container is a hermetically sealed blister-pack, in which a concave well containing a contact lens is covered by a metal or plastic sheet adapted for peeling in order to open the blister-pack.

In one embodiment, the compositions and/or solutions of the present invention may be formulated as a "multi-purpose solution" as defined in claim 16. A multi-purpose solution is useful for cleaning, disinfecting, storing, and rinsing a lens, particularly soft contact lenses. Multi-purpose solutions do not exclude the possibility that some wearers, for example, wearers particularly sensitive to chemical disinfectants or other chemical agents, may prefer to rinse or wet a contact lens with another solution, for example, a sterile saline solution prior to insertion of the lens. The term "multi-purpose solution" also does not exclude the possibility of periodic cleaners not used on a daily basis or supplemental cleaners for further removing proteins, for example, enzyme cleaners, which are typically used on a weekly basis. By the term "cleaning" is meant that the solution contains one or more agents in sufficient concentrations to loosen and remove loosely held lens deposits and other contaminants on the surface of a contact lens, which may be used in conjunction with digital manipulation (e.g., manual rubbing of the lens with a solution) or with an accessory device that agitates the solution in contact with the lens, for example, a mechanical cleaning aid.

Traditionally, multi-purpose solutions on the market have required a regimen involving mechanical rubbing of the lens with the multi-purpose solution, in order to provide the required disinfection and cleaning. Such a regimen is required under governmental regulatory authorities (e.g., the FDA or U.S. Food & Drug Administration (FDA)) for a Chemical Disinfection System that does not qualify as a Chemical Disinfecting Solution. In one embodiment of the present invention, it is possible to formulate a cleaning and disinfecting product that, on one hand, is able to provide improved cleaning and disinfection in the absence of a rubbing regimen and, on the other hand, is gentle enough to be used as a wetting agent, e.g. as an eye drop. For example, a product qualifying as a Chemical Disinfecting Solution must meet biocidal performance criteria established by the US FDA for Contact Lens Care Products (May 1, 1997) which criteria does not involve rubbing of the lenses. In one embodiment of the present invention, a composition is formulated to meet the requirements of the FDA or ISO Stand-Alone Procedure for contact lens disinfecting products. Similarly, the compositions of the present invention can be formulated to provide enhanced cleaning without the use of a rubbing regimen. Such formulations may ensure higher patient compliance and greater universal appeal than traditional multi-purpose disinfecting and cleaning products. A multi-purpose solution preferably has a viscosity of less than about 75 cps, preferably about 1 to about 50 cps, and most preferably about 1 to about 25 cps and is preferably at least about 95 percent weight by volume water in the total composition.

The aqueous ophthalmic solutions of this embodiment may contain, in addition to the copolymers described above, one or more antimicrobial agents, preservatives and the like. The compositions generally include a primary antimicrobial agent. Antimicrobial agents suitable for use in the present invention include chemicals that derive their antimicrobial activity through a chemical or physiochemical interaction with the microbial organisms. These agents may be used alone or in combination.

Suitable known ophthalmically acceptable antimicrobial agents include, but are not limited to, a biguanide or a salt or free base thereof, quaternary ammonium compound or a salt thereof or free base thereof; terpene or derivative thereof, a branched, glycerol monoalkyl ether, a branched, glycerol monoalkyl amine, a branched, glycerol monoalkyl sulphide, a fatty acid monoester, wherein the fatty acid monoester comprises an aliphatic fatty acid portion having six to fourteen carbon atoms, and an aliphatic hydroxyl portion, amidoamine compound, and the like and combinations thereof.

Suitable biguanide antimicrobial agents for use in the ophthalmic compositions of the present inventions can be any biguanide or salt thereof known in the art. Representative biguanides include non-polymeric biguanides, polymeric biguanides, salts thereof, free bases thereof and the like and mixtures thereof. Representative non-polymeric biguanides are the bis(biguanides), such as alexidine, chlorhexidine, salts of alexidine, e.g., alexidine HCl, salts of chlorhexidine, alexidine free base, and the like and mixtures thereof. The salts of alexidine and chlorhexidine can be either organic or inorganic and are typically disinfecting nitrates, acetates, phosphates, sulfates, halides and the like.

Representative polymeric biguanides include polymeric hexamethylene biguanides (PHMB) (commercially available from Zeneca, Wilmington, Del.), their polymers and water-soluble salts. In one embodiment, water-soluble polymeric biguanides for use herein can have a number average molecular weight of at least about 1,000 and more preferably a number average molecular weights from about 1,000 to about 50,000. Suitable water-soluble salts of the free bases include, but are not limited to, hydrochloride, borate, acetate, gluconate, sulfonate, tartrate and citrate salts. Generally, the hexamethylene biguanide polymers, also referred to as polyaminopropyl biguanide (PAPB), have number average molecular weights of up to about 100,000. Such compounds are known and are disclosed in U.S. Pat. No. 4,758,595 which patent is incorporated herein be reference.

PHMB is best described as a polymeric biguanide composition comprising at least three and preferably at least six biguanide polymers, which we refer to as PHMB-A, PHMB-CG and PHMB-CGA, the general chemical structures of which are depicted below.

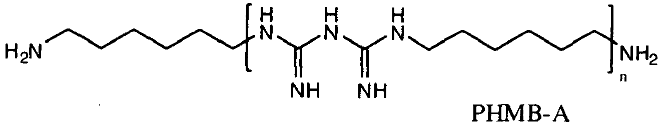

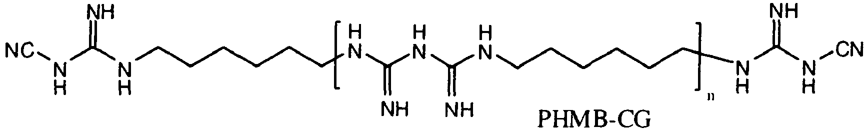

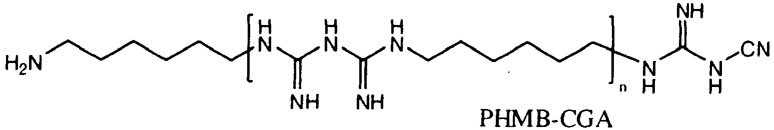

For each of these polymers, "n" represents the average number of repeating groups. Actually, a distribution of polymer length would exist for each of the polymers shown. The prior synthetic routes to PHMB provided a polymeric biguanide composition with about 50% by weight of the polymeric composition as PHMB-CGA, that is, having a cyanoguanidino end cap on one end and an amine on the other end, about 25% by weight PHMB-A and about 25% by weight PHMB-CG. Given this approximate weight ratio of the three major PHMB polymers above, the percentage of cyanoguardino end caps is also about 50% of the total number of terminal groups. In this application we refer to this conventional polymeric biguanide composition as poly(hexamethylene biguanide) or PHMB.

A new synthetic route to polymeric biguanide compositions is described in copending U.S. provisional application serial nos. 60/853,579, filed October 23, 2006, and 60/895,770, filed March 20, 2007, the entire disclosure of each of which is incorporated by reference herein. The new synthetic route provides a polymeric biguanide composition comprising less than 18 mole % of terminal amine groups as measured by ¹³CNMR. The polymeric biguanide composition can also be characterized by a relative increase in the molar concentration of terminal guanidine groups or terminal cyanoguardino groups. For example, in one embodiment, the biguanide composition comprises less than about 18 mole % of terminal amine groups and about 40 mol% or greater of terminal guanidine groups. In another embodiment, the biguanide composition comprises less than about 18 mole % of terminal amine groups and about 55 mol% or greater of terminal guanidine groups.

In this application, we refer to this biguanide composition as PHMB-CG*. We also refer to polymeric biguanide compositions in the generic sense as "hexamethylene biguanides", which one of ordinary skill in the art would recognize to include both PHMB as well as PHMB-CG*.

Representative examples of suitable quaternary ammonium compounds for use in the ophthalmic compositions of the present invention include, but are not limited to, poly[(dimethyliminio)-2-butene-1,4-diyl chloride] and [4-tris(2-hydroxyethyl)ammonio]-2-butenyl-w-[tris(2-hydroxyethyl)ammonio]-dichloride (chemical registry no. 75345-27-6) generally available as Polyquaternium 1 under the tradename ONAMER^{®} M (Stepan Company, Northfield, Ill), and the like and mixtures thereof.

Suitable terpene antimicrobial agents for use in the ophthalmic compositions of the present invention include any monoterpene, sesquiterpene and/or diterpene or derivatives thereof. Acyclic, monocyclic and/or bicyclic mono-, sesqui- and/or diterpenes, and those with higher numbers of rings, can be used. A "derivative" of a terpene as used herein shall be understood to mean a terpene hydrocarbon having one or more functional groups such as terpene alcohols, terpene ethers, terpene esters, terpene aldehydes, terpene ketones and the like and combinations thereof. Here, both the trans and also the cis isomers are suitable. The terpenes as well as the terpene moiety in the derivative can contain from 6 to about 100 carbon atoms and preferably from about 10 to about 25 carbon atoms.

Representative examples of suitable terpene alcohol antimicrobial agents include verbenol, transpinocarveol, cis-2-pinanol, nopol, isoborneol, carbeol, piperitol, thymol, α-terpineol, terpinen-4-ol, menthol, 1,8-terpin, dihydro-terpineol, nerol, geraniol, linalool, citronellol, hydroxycitronellol, 3,7-dimethyl octanol, dihydro-myrcenol, tetrahydro-alloocimenol, perillalcohol, falcarindiol and the like and mixtures thereof.

Representative examples of suitable terpene ether and terpene ester antimicrobial agents include 1,8-cineole, 1,4-cineole, isobornyl methylether, rose pyran, α-terpinyl methyl ether, menthofuran, trans-anethole, methyl chavicol, allocimene diepoxide, limonene monoepoxide, isobornyl acetate, nonyl acetate, α-terpinyl acetate, linalyl acetate, geranyl acetate, citronellyl acetate, dihydro-terpinyl acetate, meryl acetate and the like and mixtures thereof.

Representative examples of terpene aldehyde and terpene ketone antimicrobial agents include myrtenal, campholenic aldehyde, perillaldehyde, citronellal, citral, hydroxy citronellal, camphor, verbenone, carvenone, dihydro-carvone, carvone, piperitone, menthone, geranyl acetone, pseudo-ionone, α-ionine, iso-pseudo-methyl ionone, n-pseudo-methyl ionone, iso-methyl ionone, n-methyl ionone and the like amd mixtures thereof. Any other terpene hydrocarbons having functional groups known in the art may be used herein in the inventive composition.

In one embodiment, suitable terpenes or derivatives thereof as antimicrobial agents include, but are not limited to, tricyclene, α-pinene, terpinolene, carveol, amyl alcohol, nerol, β-santalol, citral, pinene, nerol, b-ionone, caryophillen (from cloves), guaiol, anisaldehyde, cedrol, linalool, d-limonene (orange oil, lemon oil), longifolene, anisyl alcohol, patchouli alcohol, α-cadinene, 1,8-cineole, ρ-cymene, 3-carene, ρ-8-mentane, trans-menthone, borneol, α-fenchol, isoamyl acetate, terpin, cinnamic aldehyde, ionone, geraniol (from roses and other flowers), myrcene (from bayberry wax, oil of bay and verbena), nerol, citronellol, carvacrol, eugenol, carvone, α-terpineol, anethole, camphor, menthol, limonene, nerolidol, farnesol, phytol, carotene (vitamin A₁), squalene, thymol, tocotrienol, perillyl alcohol, borneol, simene, carene, terpenene, linalool, 1-terpene-4-ol, zingiberene (from ginger) and the like and mixtures thereof.

In one embodiment, the compound of component (ii) of the ophthalmic composition comprises a branched, glycerol monoalkyl ether. In another embodiment, the compound of component (ii) of the ophthalmic composition comprises a branched, glycerol monoalkyl amine. In another embodiment, the compound of component (ii) of the ophthalmic composition comprises a branched, glycerol monoalkyl sulphide. In still another embodiment, the compound of component (ii) of the ophthalmic composition comprises any one mixture of a branched, glycerol monoalkyl ether, a branched, glycerol monoalkyl amine or a branched, glycerol monoalkyl sulphide.

In one embodiment, the branched, glycerol monoalkyl ether for use in the ophthalmic compositions of the present invention is 3-[(2-ethylhexyl)oxy]-1,2-propanediol (EHOPD). In another embodiment, the branched, glycerol monoalkyl amine is 3-[(2-ethylhexyl)amino]-1,2-propanediol (EHAPD). In another embodiment, the branched, glycerol monoalkyl sulphide is 3-[(2-ethylhexyl)thio]-1,2-propanediol (EHSPD). In still another embodiment, the ophthalmic composition comprises any one mixture of EHOPD, EHAPD and EHSPD. The chemical structures of EHOPD, EHAPD and EHSPD are provided below.

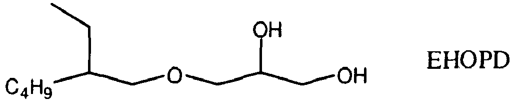

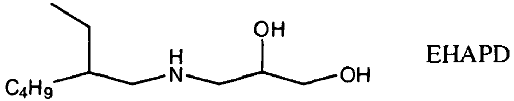

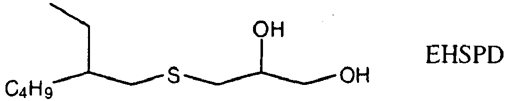

EHOPD is also referred to as octoxyglycerin and is sold under the tradename Sensiva® SC50 (Schülke & Mayr). EHOPD is a branched, glycerol monoalkyl ether known to be gentle to the skin, and to exhibit antimicrobial activity against a variety of Gram-positive bacteria such as *Micrococcus luteus, Corynebacterium aquaticum, Corynebacterium flavescens, Corynebacterium callunae, and Corynebacterium nephredi.* Accordingly, EHOPD is used in various skin deodorant preparations at concentrations between about 0.2 and 3 percent by weight. EHAPD can be prepared from 2-ethylhexylamine and 2,3-epoxy-1-propanediol using chemistry well known to those of ordinary skill in the art. EHSPD can be prepared from 2-ethylhexylthiol and 2,3-epoxy-1-propanediol using chemistry well known to those of ordinary skill in the art.

Suitable fatty acid monoester for use in the ophthalmic compositions of the present invention include those fatty acid monoesters comprising an aliphatic fatty acid portion having six to fourteen carbon atoms, and an aliphatic hydroxyl portion.

The term "aliphatic" refers to a straight or branched, saturated or unsaturated hydrocarbon having six to fourteen carbon atoms. In one embodiment, the aliphatic fatty acid portion is a straight chain, saturated or unsaturated hydrocarbon with eight to ten carbons. In another embodiment, the aliphatic fatty acid portion is a branched chain, saturated or unsaturated hydrocarbon with eight to ten carbons.

The aliphatic hydroxyl portion of the fatty acid monoester can be any aliphatic compound with at least one hydroxyl group. In many of the embodiments, the aliphatic hydroxyl portion will have from three to nine carbons. The aliphatic hydroxyl portion can include, but is not limited to, propylene glycol, glycerol, a polyalkylene glycol, e.g., polyethylene glycol or polypropylene glycol, a cyclic polyol, e.g., sorbitan, glucose, mannose, sucrose, fructose, fucose and inisitol and derivatives thereof, and a linear polyol, e.g., mannitol and sorbitol and derivatives thereof and the like and mixtures thereof.

Representative examples of suitable amidoamines for use in the ophthalmic compositions of the present inventions include those amidoamines of the general formula:

R¹²-(OCH₂CH₂)ₘ-X-(CH₂)ₙ-Y

wherein R¹² is a is C₆-C₃₀ saturated or unsaturated hydrocarbon including by way of example, a straight or branched, substituted or unsubstituted alkyl, alkylaryl, or alkoxyaryl group ; m is zero to 16; n is 2 to 16; X is -C(O)-NR¹³- or -R¹³N-C(O)-;Y is -N(R¹⁴)₂ wherein each of R¹³ and R¹⁴ independently are hydrogen, a C₁-C₈ saturated or unsaturated alkyl or hydroxyalkyl, or a pharmaceutically acceptable salt thereof. In one embodiment, m is 0, R¹² is heptadec-8-enyl, undecyl, undecenyl, dodecyl, tridecyl, tetradecyl, pentadecyl or heptadecyl, R² is hydrogen or methyl, and R³ is methyl or ethyl.

Some of the amidoamines utilized in the present invention are available from commercial sources. For example, myristamidopropyl dimethylamine is available from Alcon Inc. (Fort Worth, Tx.) under the tradename Aldox^{®}; lauramidopropyl dimethylamine is available from Inolex Chemical Company (Philadelphia, Pa.) under the tradename LEXAMlNE^{®} L-13; and stearamidopropyl dimethylamine is also from Inolex Chemical Company as LEXAMINE^{®} S-13. The above-described amidoamines can be synthesized in accordance with known techniques, including those described in U.S. Patent No. 5,573,726.

The amount of the primary antimicrobial agent may vary depending on the specific agent employed. For the aforementioned organic nitrogen-containing agent, typically, such agents are present in concentrations ranging from about 0.00001 to about 0.5% weight percent, and more preferably, from about 0.00003% to about 0.05% weight percent. For sorbic acid, higher amounts may be required, typically about 0.01 to about 1 weight percent, more preferably about 0.1 to about 0.5 weight percent. It is preferred that the antimicrobial agent is used in an amount that will at least partially reduce the microorganism population in the formulations employed. If desired, the antimicrobial agent may be employed in a disinfecting amount, which will reduce the microbial bioburden by at least two log orders in four hours and more preferably by one log order in one hour. Most preferably, a disinfecting amount is an amount which will eliminate the microbial burden on a contact lens when used in regimen for the recommended soaking time (FDA Chemical Disinfection Efficacy Test-July, 1985 Contact Lens Solution Draft Guidelines).

The aqueous solutions of this embodiment may further contain one or more other components that are commonly present in ophthalmic solutions, for example, surfactants, tonicity adjusting agents; buffering agents; chelating agents; pH adjusting agents, viscosity modifying agents, and demulcents and the like as discussed hereinabove, and which aid in making ophthalmic compositions more comfortable to the user and/or more effective for their intended use.

The pH of the solutions and/or compositions of the present invention may be maintained within the range of pH of about 4.0 to about 9.0, preferably about 5.0 to about 8.0, more preferably about 6.0 to about 8.0, and even more preferably about 6.5 to about 7.8. In one embodiment, pH values of greater than or equal to about 7 are most preferred.

According to one embodiment of the present invention a method of treating a biomedical device involves contacting a biomedical device with a solution as defined in claim 12. . In an embodiment, the method involves immersing the biomedical device in the solution removing the device from the solution; packaging the biomedical device in a packaging solution in a manner preventing contamination of the device by microorganisms; and sterilizing the packaged solution and device. According to this embodiment, the packaging solution can be any packaging solution known in the art or, alternatively, a packaging solution containing a polymerization product as described herein which contains (a) a monomer bearing a center of permanent positive charge and/or (b) a non-ionic ethylenically unsaturated monomer different than the solution in which the biomedical device was first immersed. In yet another embodiment, the method involves immersing the biomedical device in a solution as defined according to the present invention; packaging the solution containing the biomedical device in a packaging solution in a manner preventing contamination of the device by microorganisms; and sterilizing the packaged solution and device. In this embodiment, the solution containing the biomedical device can be stored "as is" in a packaging solution known in the art or, alternatively, in a packaging solution containing a polymerization product as described herein which contains (a) a monomer bearing a center of permanent positive charge and/or (b) a non-ionic ethylenically unsaturated monomer different than the solution in which the biomedical device was immersed.

In still yet another embodiment, the method involves immersing the biomedical device in a solution as defined according to the present invention; packaging the solution and the biomedical device in a manner preventing contamination of the device by microorganisms; and sterilizing the packaged solution and device. In yet another embodiment, the method involves soaking the biomedical device in a solution as defined according to the present invention; removing the biomedical device from the solution; and placing the biomedical device directly in the eye for non-therapeutic use.

As used herein, the term "biomedical device" or "ophthalmic device" refers to devices that reside in or on the eye. These devices can provide optical correction, wound care, drug delivery, diagnostic functionality or cosmetic enhancement or effect or a combination of these properties. Representative examples of such devices include, but are not limited to, ophthalmic lenses such as soft contact lenses, e.g., a soft, hydrogel lens; soft, non-hydrogel lens and the like, hard contact lenses, e.g., a hard, gas permeable lens material and the like, intraocular lenses, overlay lenses, ocular inserts, optical inserts and the like. As is understood by one skilled in the art, a lens is considered to be "soft" if it can be folded back upon itself without breaking. Any material known to produce a biomedical device including a contact lens can be used herein. It is particularly useful to employ biocompatible materials herein including both soft and rigid materials commonly used for ophthalmic lenses, including contact lenses. The preferred substrates are hydrogel materials, including silicone hydrogel materials and non-silicone hydrogel materials.

A wide variety of materials can be used herein. Hydrogels in general are a well-known class of materials that comprise hydrated, crosslinked polymeric systems containing water in an equilibrium state. Hydrogel contact lens materials are made from at least one hydrophilic monomer, such as 2-hydroxethyl methacrylate (HEMA), N-vinylpyrrolidone (NVP) or N,N-dimethylacrylamide (DMA). Hydrogels generally have a water content greater than about 15 weight percent and more commonly between about 20 to about 80 weight percent.

One class of hydrogels is silicone hydrogels. These materials are usually prepared by polymerizing a mixture containing at least one silicone-containing monomer and at least one hydrophilic monomer. Typically, either the silicone-containing monomer or the hydrophilic monomer functions as a crosslinking agent (a crosslinker being defined as a monomer having multiple polymerizable functionalities) or a separate crosslinker may be employed. Applicable silicone-containing monomeric units for use in the formation of silicone hydrogels are well known in the art and numerous examples are provided in U.S. Patent Nos. 4,136,250; 4,153,641; 4,740,533; 5,034,461; 5,070,215; 5,260,000; 5,310,779; and 5,358,995.

Representative examples of applicable silicon-containing monomeric units include bulky siloxanyl monomers represented by the structure of Formula V: 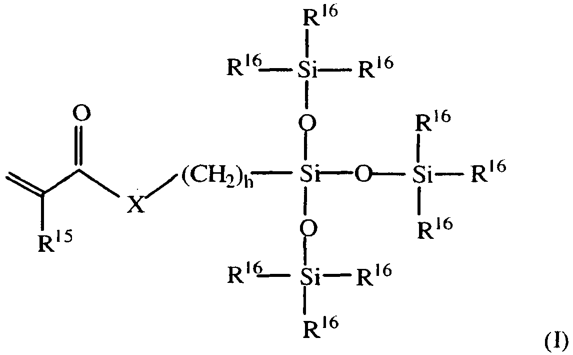 wherein X denotes -O- or -NR¹⁷-; each R¹⁵ independently denotes hydrogen or methyl; each R¹⁶ independently denotes a lower alkyl radical, phenyl radical or a group represented by 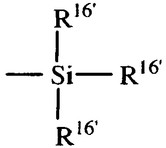 wherein each R^{16'} independently denotes a lower alkyl or phenyl radical; and h is 1 to 10.

Examples of bulky monomers are 3-methacryloyloxypropyltris(trimethyl-siloxy)silane or tris(trimethylsiloxy)silylpropyl methacrylate, sometimes referred to as TRIS and tris(trimethylsiloxy)silylpropyl vinyl carbamate, sometimes referred to as TRIS-VC and the like.

Such bulky monomers may be copolymerized with a silicone macromonomer, such as a poly(organosiloxane) capped with an unsaturated group at two or more ends of the molecule. U.S. Patent No. 4,153,641 discloses, for example, various unsaturated groups such as acryloyloxy or methacryloyloxy groups.

Another class of representative silicone-containing monomers includes, but is not limited to, silicone-containing vinyl carbonate or vinyl carbamate monomers such as, for example, 1,3-bis[4-vinyloxycarbonyloxy)but-1-yl]tetramethyl-disiloxane; 3-(trimethylsilyl)propyl vinyl carbonate: 3-(vinyloxycarbonylthio)propyl-[tris(trimethylsiloxy)silane]; 3-[tris(trimethylsiloxy)silyl]propyl vinyl carbamate; 3-[tris(trimethylsiloxy)silyl]propyl allyl carbamate; 3-[tris(trimethylsiloxy)silyl]propyl vinyl carbonate; t-butyldimethylsiloxyethyl vinyl carbonate; trimethylsilylethyl vinyl carbonate; trimethylsilylmethyl vinyl carbonate and the like and mixtures thereof.

Another class of silicon-containing monomers includes polyurethane-polysiloxane macromonomers (also sometimes referred to as prepolymers), which may have hard-soft-hard blocks like traditional urethane elastomers. They may be end-capped with a hydrophilic monomer such as 2-hydroxyethyl methacrylate (HEMA). Examples of such silicone urethanes are disclosed in a variety or publications, including PCT Published Application No. WO 96/31792 discloses examples of such monomers . Representative examples of silicone urethane monomers are represented by Formulae VI and VII:

E(*D*A*D*G)ₐ *D*A*D*E'; (II)

or

E(*D*G*D*A)ₐ *D*A*D*E'; (III)

or
wherein:
D independently denotes an alkyl diradical, an alkyl cycloalkyl diradical, a cycloalkyl diradical, an aryl diradical or an alkylaryl diradical having 6 to about 30 carbon atoms;
G independently denotes an alkyl diradical, a cycloalkyl diradical, an alkyl cycloalkyl diradical, an aryl diradical or an alkylaryl diradical having 1 to about 40 carbon atoms and which may contain ether, thio or amine linkages in the main chain;
* denotes a urethane or ureido linkage;
a is at least 1;

A independently denotes a divalent polymeric radical of Formula VIII: 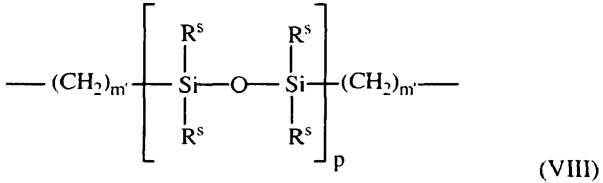
wherein each R^{s} independently denotes an alkyl or fluoro-substituted alkyl group having 1 to about 10 carbon atoms which may contain ether linkages between the carbon atoms; m' is at least 1; and p is a number that provides a moiety weight of about 400 to about 10,000;
each of E and E' independently denotes a polymerizable unsaturated organic radical represented by Formula IX: 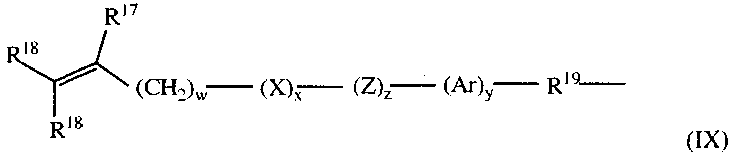 wherein: R¹⁷ is hydrogen or methyl;
   R¹⁸ independently is hydrogen, an alkyl radical having 1 to 6 carbon atoms, or a -COY-R²⁰ radical wherein Y is -O-, -S- or -NH-;
   R¹⁹ is a divalent alkylene radical having 1 to about 10 carbon atoms;
   R²⁰ is a alkyl radical having 1 to about 12 carbon atoms;
   X denotes -CO- or -OCO-;
   Z denotes -O- or -NH-;
   Ar denotes an aromatic radical having about 6 to about 30 carbon atoms;
   w is 0 to 6; x is 0 or 1; y is 0 or 1; and z is 0 or 1.

A preferred silicone-containing urethane monomer is represented by Formula X: 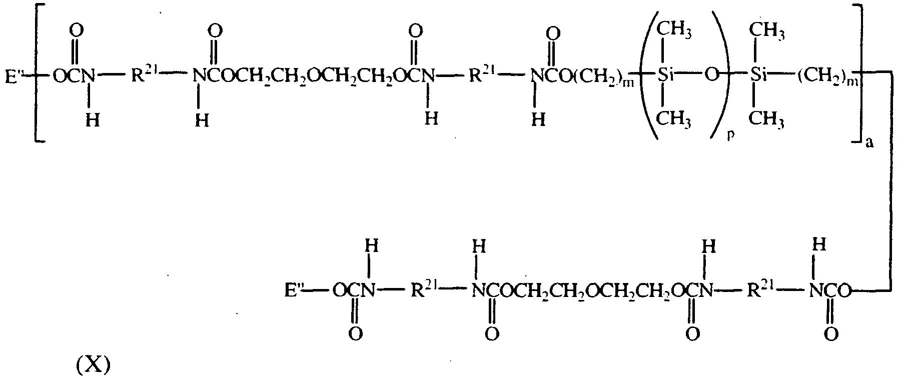 wherein m is at least 1 and is preferably 3 or 4, a is at least 1 and preferably is 1, p is a number which provides a moiety weight of about 400 to about 10,000 and is preferably at least about 30, R²¹ is a diradical of a diisocyanate after removal of the isocyanate group, such as the diradical of isophorone diisocyanate, and each E" is a group represented by:

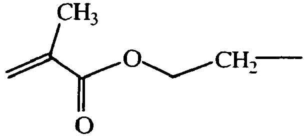

In another embodiment of the present invention, a silicone hydrogel material comprises (in bulk, that is, in the monomer mixture that is copolymerized) about 5 to about 50 percent, and preferably about 10 to about 25, by weight of one or more silicone macromonomers, about 5 to about 75 percent, and preferably about 30 to about 60 percent, by weight of one or more polysiloxanylalkyl (meth)acrylic monomers, and about 10 to about 50 percent, and preferably about 20 to about 40 percent, by weight of a hydrophilic monomer. In general, the silicone macromonomer is a poly(organosiloxane) capped with an unsaturated group at two or more ends of the molecule. In addition to the end groups in the above structural formulas, U.S. Patent No. 4,153,641 discloses additional unsaturated groups, including acryloyloxy or methacryloyloxy groups. Fumarate-containing materials such as those disclosed in U.S. Patent Nos. 5,310,779; 5,449,729 and 5,512,205 are also useful substrates in accordance with the invention. Preferably, the silane macromonomer is a silicon-containing vinyl carbonate or vinyl carbamate or a polyurethane-polysiloxane having one or more hard-soft-hard blocks and end-capped with a hydrophilic monomer.

Suitable hydrophilic monomers include amides such as N,N-dimethylacrylamide and N,N-dimethylmethacrylamide, cyclic lactams such as N-vinyl-2-pyrrolidone and poly(alkene glycols) functionalized with polymerizable groups. Examples of useful functionalized poly(alkene glycols) include poly(diethylene glycols) of varying chain length containing monomethacrylate or dimethacrylate end caps. In a preferred embodiment, the poly(alkene glycol) polymer contains at least two alkene glycol monomeric units. Still further examples are the hydrophilic vinyl carbonate or vinyl carbamate monomers disclosed in U.S. Patent No. 5,070,215, and the hydrophilic oxazolone monomers disclosed in U.S. Patent No. 4,910,277. Other suitable hydrophilic monomers will be apparent to one skilled in the art.

In one embodiment, the lens can be a Group II and Group IV lens having a water content greater than about 50% by weight, and preferably about 55% to about 80% water, although the invention is applicable for any type of soft hydrogel contact lens. Representative contact lens materials include, but are not limited to materials known by the following USAN and the USAP Dictionary of Drug Names: bufilcon A, etafilcon A, methafilcon A, ocufilcon C, perfilcon A, phemfilcon A, vifilcon A, hilafilcon A, hilafilcon B, balafilcon A, methafilcon B, ocufilcon D, methafilcon A, etafilcon A lidofilcon A or B, and alphafilcon A.

The above materials are merely exemplary, and other materials for use as substrates that can benefit by being cleaned and disinfected with the ophthalmic compositions according to the present invention and have been disclosed in various publications and are being continuously developed for use in ophthalmic devices such as contact lenses and other medical devices can also be used. For example, an ophthalmic device for use herein can be an ophthalmic lens such as a contact lens or the ophthalmic device can be fluorinated silicone-containing monomers. Such monomers have been used in the formation of fluorosilicone hydrogels to reduce the accumulation of deposits on contact lenses made therefrom, as disclosed in, for example, U.S. Patent Nos. 4,954,587; 5,010,141 and 5,079,319. The use of silicone-containing monomers having certain fluorinated side groups, i.e., -(CF₂)-H, have been found to improve compatibility between the hydrophilic and silicone-containing monomeric units. See, e.g., U.S. Patent Nos. 5,321,108 and 5,387,662.

Ophthalmic devices such as contact lenses for application of the present invention can be manufactured employing various conventional techniques, to yield a shaped article having the desired posterior and anterior lens surfaces. Spincasting methods are disclosed in U.S. Patent Nos. 3,408,429 and 3,660,545; preferred static casting methods are disclosed in U.S. Patent Nos. 4,113,224 and 4,197,266. Curing of the monomeric mixture is often followed by a machining operation in order to provide a contact lens having a desired final configuration. As an example, U.S. Patent No. 4,555,732 discloses a process in which an excess of a monomeric mixture is cured by spincasting in a mold to form a shaped article having an anterior lens surface and a relatively large thickness. The posterior surface of the cured spincast article is subsequently lathe cut to provide a contact lens having the desired thickness and posterior lens surface. Further machining operations may follow the lathe cutting of the lens surface, for example, edge-finishing operations.

After producing a lens having the desired final shape, it is desirable to remove residual solvent from the lens before edge-finishing operations. This is because, typically, an organic diluent is included in the initial monomeric mixture in order to minimize phase separation of polymerized products produced by polymerization of the monomeric mixture and to lower the glass transition temperature of the reacting polymeric mixture, which allows for a more efficient curing process and ultimately results in a more uniformly polymerized product. Sufficient uniformity of the initial monomeric mixture and the polymerized product are of particular concern for silicone hydrogels, primarily due to the inclusion of silicone-containing monomers which may tend to separate from the hydrophilic comonomer. Suitable organic diluents include, for example, monohydric alcohols such as C₆-C₁₀ straight-chained aliphatic monohydric alcohols, e.g., n-hexanol and n-nonanol; diols such as ethylene glycol; polyols such as glycerin; ethers such as diethylene glycol monoethyl ether; ketones such as methyl ethyl ketone; esters such as methyl enanthate; and hydrocarbons such as toluene. Preferably, the organic diluent is sufficiently volatile to facilitate its removal from a cured article by evaporation at or near ambient pressure. Generally, the diluent is included at about 5 to about 60 percent by weight of the monomeric mixture, with about 10 to about 50 percent by weight being especially preferred.

The cured lens is then subjected to solvent removal, which can be accomplished by evaporation at or near ambient pressure or under vacuum. An elevated temperature can be employed to shorten the time necessary to evaporate the diluent. The time, temperature and pressure conditions for the solvent removal step will vary depending on such factors as the volatility of the diluent and the specific monomeric components, as can be readily determined by one skilled in the art. According to a preferred embodiment, the temperature employed in the removal step is preferably at least about 50°C, for example, about 60°C to about 80°C. A series of heating cycles in a linear oven under inert gas or vacuum may be used to optimize the efficiency of the solvent removal. The cured article after the diluent removal step should contain no more than twenty percent by weight of diluent, preferably no more than about 5 percent by weight or less.

Following removal of the organic diluent, the lens can then be subjected to mold release and optional machining operations. The machining step includes, for example, buffing or polishing a lens edge and/or surface. Generally, such machining processes may be performed before or after the article is released from a mold part. Preferably, the lens is dry released from the mold by employing vacuum tweezers to lift the lens from the mold, after which the lens is transferred by means of mechanical tweezers to a second set of vacuum tweezers and placed against a rotating surface to smooth the surface or edges. The lens may then be turned over in order to machine the other side of the lens

The following examples are provided to enable one skilled in the art to practice the invention and are merely illustrative of the invention. The examples should not be read as limiting the scope of the invention as defined in the claims.

In the examples, the following abbreviations are used.
V2 D25: pentacontamethyl-α,ω-bis (4-vinyloxycarbonyloxybutyl)pentacosa siloxane (Mw range 2-4k)
TRISVC: 3-[tris(tri-methylsiloxy)silyl]propyl vinyl carbamate
NVP: N-vinyl-2-pyrrolidone
D1173: 2-hydroxy-2-methyl-1-phenylpropan-1-one (available as Darocur 1173 initiator)
IMVT: 1,4-bis(4-(2-methacryloxyethyl)phenylamino)anthraquinone

### EXAMPLE 1

### Preparation of a silicone hydrogel lens.

A monomer mix was made by mixing the following components, listed in Table 1 at amounts per weight.

**TABLE 1**

| Ingredient | Amount |
|---|---|
| V2 D25 | 15 |
| TRISVC | 55 |
| Vinal acid | 1 |
| NVP | 30 |
| Nonanol | 15 |
| Darocur-1173 | 0.5 |
| | |
| IMVT | 100 ppm |

The resultant monomer mixture was cast into contact lenses by introducing the monomer mixture to a mold assembly composed of two polypropylene mold sections, the front mold section having a mold surface for forming a front contact lens surface and the back mold section having a mold surface for forming a back contact lens surface. Then, the mold section and monomer mixture were exposed to ultraviolet light to induce free radical polymerization and cure the monomer mixture to form a contact lens. The resultant contact lenses were removed from the mold assembly, extracted in an oven at 60°C for an hour, and the dried lenses were released from the molds.

### EXAMPLE 2

Post-treatment of the lenses of Example 1 with a copolymer of NVP and dimethylaminoethyl methacrylate, quaternized with diethyl sulfate obtained from Aldrich Chemical Company (Milwaukee, Wisconsin) as a 20 % solution in water.

A control lens was prepared by plasma treating the lenses of Example 1 with ammonia gas followed by extraction with isopropanol for 2 hours, hydrated in DI water and then autoclaved (121°C) in borate buffered saline (control lenses). A test lens according to the present invention was prepared by plasma treating the lenses of Example 1 with ammonia gas followed by extraction in a solution made from a mixture of DI water (70 mL), acrylic acid (13.12 g), NaOH (8.526 g) and isopropanol (350 ml) overnight, hydrated in DI water containing 30 ppm of copolymer of NVP and dimethylaminoethylmethacrylate, quaternized with diethyl sulfate (Aldrich Chemical Company) overnight, saved in a borate buffered saline and then autoclaved (121°C) (test lenses).

### Tests:

The control and test lenses of Example 2 were characterized by contact angle measurement and XPS.

### I. Contact Angle Measurement

The contact angle measurement for the control and test lenses prepared in Example 2 was carried out as follows:
The lenses were placed in polystyrene Petri dishes containing HPLC grade water for 15 minutes. The lenses were quartered using a clean scalpel. The quarters were mounted on a clean glass slide and dried overnight in a nitrogen dry-box. The contact angles were measured on the dehydrated lenses at two points on each quarter.

The instrument used for the measurement was an AST Products Video Contact Angle System (VCA) 2500XE. This instrument utilizes a low power microscope tat produces a sharply defined image of the water drop, which is captured immediately on the computer screen. HPLC water was drawn into the VCA system microsyringe, and a 0.6µl drop is dispensed from the syringe onto the sample. The contact angle is calculated by placing three to five markers along the circumference of the drop and the contact angle is recorded. Both a right and left angle are reported for each measurement and an average was calculated and recorded. The results of this test are set forth below in Table 2.

**TABLE 2**

| Lens | Contact Angle (degrees) |
|---|---|
| Control | 113 |
| Test (autoclaved) | 46.3 |

The sharp drop in the contact angle indicated that the lens surface became very wettable after treatment with the cationic NVP copolymer.

### II. XPS Analysis

The control and test lenses of Example 2 were analyzed for their atomic concentration by X-ray Photoelectron Spectrometer (XPS). The XPS utilized in this study was a Physical Electronics [PHI] Model 5600. This instrument utilized an aluminum anode operated at 300 watts, 15 kV and 27 milliamps. The excitation source was monochromatized utilizing a torodial lens system. The 7 mm filament was utilized for the polymer analysis due to the reduced sample damage and ease of photoionization neutralization. The base pressure of this instrument was 2.0x10⁻¹⁰ torr while the pressure during operation was 1.0x10⁻⁹ torr. This instrument made use of a hemispherical energy analyzer. The practical measure of sampling depth for this instrument at a sampling angle of 45° and with respect to carbon was approximately 74 angstroms. All elements were charge corrected to the CHₓ peak of carbon to a binding energy of 285.0 electron volts (eV).

Each of the specimens was analyzed utilizing a low resolution survey spectra [0-1100 eV] to identify the elements present on the sample surface. The high resolution spectra were performed on those elements detected from the low resolution scans. The elemental composition was determined from the high resolution spectra. The atomic composition was calculated from the areas under the photoelectron peaks after sensitizing those areas with the instrumental transmission function and atomic cross sections for the orbital of interest. Since XPS does not detect the presence of hydrogen or helium, these elements will not be included in any calculation of atomic percentages. It is also noted that atomic percentages may vary if a different instrument design, i.e., transmission function, is utilized, so that for purposes of exact reproducibility the atomic percentage numbers in the application refer to the specified instrument design, as will be understood by the skilled artisan.

The low resolution XPS survey spectra taken at a takeoff angle of 45° for the untreated lens' surfaces contained peaks for carbon, nitrogen, oxygen, boron and sodium. The analysis of the lens' material begins with the examination of the unmodified matrix (control). Table I below contains the XPS data for the samples. This data reflects the atomic composition over the top 74 angstroms (relative to carbon Is electrons). The XPS results for the lenses of Example 2 are set forth below in Table 3.

**TABLE 3**

| | | | | |
|---|---|---|---|---|
| | Atomic Concentration | | | |
| | C1s | N1s | O1s | Si2p |
| Control lens | 64.2(3.8) | 7.4(0.5) | 19.4(0.4) | 9.0(0.8) |
| Test lens (non-autoclaved) | 66.7(0.5) | 10.0(1.4) | 20.7(1.2) | 2.6(0.4) |
| Test lens (autoclaved) | 67.0(0.7) | 10.2(0.5) | 20.0(0.6) | 2.9(0.6) |

As the data show, the lens surface coated with the cationic NVP copolymer resulted in a sharp decrease in silicon atom content and increase in nitrogen content, indicating that the lens surface became very polar after the treatment, and therefore more wettable, less lipid-like deposits and more comfortable to wear.

### EXAMPLE 3

### Preparation of a copolymer of 3-methacryloylaminopropyl-dimethyl(3-sulfopropyl)ammonium hydrochloride (MAPSA) and glyceryl methacrylate (GM).

MAPSA (0.925 g, 3.163 mmole, from Aldrich Chemical Company) was dissolved in 50 ml of deionized water. Into a 250 ml three-neck flask equipped with a stirrer bar connected to a condenser which was connected to an oil bubbler was added deionized water (150 ml), GM (4.963 g, 31.02 mmole) via a syringe, the MAPSA solution and azo bis-isobutylnitrile (AIBN, 0.050 g;). The reaction mixture was vigorously bubbled with nitrogen for 20 minutes under stirring and then nitrogen flow was turned lower. The reaction mixture was heated with an oil bath to 70°C and held at this temperature for 48 hours under a constant nitrogen purge. The product was saved in DI water

### EXAMPLE 4

### Surface treatment of contact lens.

A borated buffer saline solution is prepared containing 1% part of the copolymer of Example 3. The borated buffer saline solution is filled into a blister pack and negatively charged silicone hydrogel contact lenses sold by Bausch & Lomb under the trade name Pure Vision^{®} are placed into the solution. The blister packs are then sealed and autoclaved for one cycle (e.g., at 121°C).

### EXAMPLE 5

### Preparation of a copolymer of 2-methacryloxyethyl phosphorylcholine and N-vinyl-2-pyrrolidone (NVP).

The copolymer of this example may be prepared according to the procedure of Example 3, employing the following components: deionized water (100 ml), NVP (11.1 g; 100 mmole), 2-methacryloxyethyl phosphorylcholine (4.4 g) and azo bis-isobutylnitrile (AIBN) (0.092 g; 0.56 mmol).

### EXAMPLE 6

### Preparation of a copolymer of 2-methacryloxyethyl phosphorylcholine and GM.

The copolymer of this example may be prepared according to the procedure of Example 3, employing the following components: deionized water (100 ml), GM (16 g; 100 mmole), 2-methacryloxyethyl phosphorylcholine (4.4 g) and AIBN (0.092 g; 0.56 mmol).

### EXAMPLE 7

### Preparation of a copolymer of (3-methacryloylamino)propyl trimethyl ammonium chloride and GM.

The copolymer of this example may be prepared according to the procedure of Example 3, employing the following components: deionized water (100 ml), GM (16 g; 100 mmole), (3-methacryloylamino)propyl trimethyl ammonium chloride (4.4 g, 2mmole) and AIBN (0.092 g; 0.56 mmol).

### EXAMPLE 8

### Dehydration Test

The control and test lenses prepared in Example 2 were subjected to a dehydration test. The dehydration test procedure is described as follows.

The control and test lenses were desalinated, placed in deionized water before being evaluated for dehydration. Dehydration tests were carried out using a TA Instrument Q50 thermal gravimetric analyzer ("TGA"). A disc of approximately 7 mm in diameter was punched from the center of a lens. The disc was dabbed with Kimwipes^{®} to remove any surface water and then placed on the TGA balance. The balance was enclosed in a chamber under a dry nitrogen pure (at 60 ml/minute flow rate). The sample was ramped at 50°C/minute up to 37°C and held isothermally. Mass loss versus time was monitored and the test was terminated when the mass loss rate (in %/minute) was less than 0.05. The dehydration rate was calculated as rate of mass loss between 60 and 20 percent per minute and reported as mg/minute. It was found that treated lenses of Example 2 (test lenses) showed a lower rate of water loss as compared to the untreated lens (control lenses) indicating that the treated lens was more capable of holding water. In this manner, the treated lens had better wettability and was more lubricious which would result in less problems associated with dry eye.

It will be understood that various modifications may be made to the embodiments disclosed herein. Therefore the above description should not be construed as limiting, but merely as exemplifications of preferred embodiments. For example, the functions described above and implemented as the best mode for operating the present invention are for illustration purposes only.

## Claims

1. A packaging system for the storage of an ophthalmic device comprising a sealed container containing one or more unused ophthalmic device immersed in an aqueous packaging solution comprising a polymerization product obtained from a monomer mixture consisting of (a) a zwitterionic monomer bearing a center of permanent positive charge and (b) a non-ionic ethylenically unsaturated monomer, wherein the solution has an osmolality of at least about 200 mOsm/kg, a pH of about 4 to about 9 and is heat sterilized,
wherein the non-ionic ethylenically unsaturated monomer is selected from the group consisting of a hydroxyalkyl (alk)acrylate, a hydroxyalkyl acrylamide, a N-vinyl lactam, a polyhydroxyl (alk)acrylate, a polyhydroxyl acrylamide and mixtures thereof.

2. The packaging system of Claim 1, wherein the zwitterionic monomer comprises a quaternary ammonium moiety and a phosphate moiety.

3. The packaging system of Claim 1, wherein the zwitterionic monomer is selected from the group consisting of 2-methacryloxyethyl phosphorylcholine, 1-(4(4'-vinylbenzyloxy)butane)-2'(trimethylammonium)ethyl phosphate, [3-(methacryloylamino)propyl]dimethyl(3-sulfopropyl) ammonium hydroxide salt , dimethylaminoethyl methacrylate diethylsulfate and mixtures thereof.

4. The packaging system of Claim 1, wherein the polymerization product is present in the solution in an amount of about 0.005% w/w to about 1% w/w.

5. The packaging system of Claim 1, wherein the solution further comprises a buffering agent.

6. The packaging system of Claim 5, wherein the buffering agent comprises a borate or phosphate compound.

7. The packaging system of Claim 1, wherein package is heat sterilized subsequent to sealing of the package.

8. The packaging system of Claim 1, wherein the solution does not contain an effective disinfecting amount of a disinfecting agent.

9. The packaging system of Claim 1, wherein the solution does not contain a germicide compound.

10. The packaging system of Claim 1, wherein the ophthalmic device is a contact lens.

11. The packaging system of Claim 1, wherein the ophthalmic device is an anionic contact lens.

12. A method of treating a biomedical device, the method comprising:
contacting a biomedical device with a solution comprising a polymerization product obtained from a monomer mixture consisting of (a) a zwitterionic monomer bearing a center of permanent positive charge and (b) a non-ionic ethylenically unsaturated monomer,
wherein the non-ionic ethylenically unsaturated monomer is selected from the group consisting of a hydroxyalkyl (alk)acrylate, a hydroxyalkyl acrylamide, a N-vinyl lactam, a polyhydroxyl (alk)acrylate, a polyhydroxyl acrylamide and mixtures thereof.

13. The method of Claim 12, comprising:
immersing the biomedical device in the solution;
packaging the solution containing the biomedical device in a packaging solution in a manner preventing contamination of the device by microorganisms; and
sterilizing the packaged solution and device.

14. The method of Claim 12, comprising:
soaking the biomedical device in the solution;
removing the biomedical device from the solution; and
placing the biomedical device directly in the eye.

15. The method of Claim 12, wherein the biomedical device is a contact lens.

16. A multi-purpose ophthalmically acceptable solution comprising (a) a polymerization product obtained from a monomer mixture consisting of a zwitterionic monomer bearing a center of permanent positive charge and a non-ionic ethylenically unsaturated monomer; and (b) an ophthalmically acceptable carrier for the polymerization product,
wherein the non-ionic ethylenically unsaturated monomer is selected from the group consisting of a hydroxyalkyl (alk)acrylate, a hydroxyalkyl acrylamide, a N-vinyl lactam, a polyhydroxyl (alk)acrylate, a polyhydroxyl acrylamide and mixtures thereof.

17. The multi-purpose ophthalmically acceptable solution of Claim 16, wherein the zwitterionic monomer comprises a quaternary ammonium moiety and a phosphate moiety.

18. The multi-purpose ophthalmically acceptable solution of Claim 16, wherein the zwitterionic monomer is selected from the group consisting of 2-methacryloxyethyl phosphorylcholine, 1-(4(4'-vinylbenzyloxy)butane)-2'(trimethylammonium)ethyl phosphate, [3-(methacryloylamino)propyl]dimethyl(3-sulfopropyl) ammonium hydroxide salt, dimethylaminoethyl methacrylate diethylsulfate and mixtures thereof.

19. The multi-purpose ophthalmically acceptable solution of Claim 16, further comprising one or more antimicrobial agents.

## Patentansprüche

1. Ein Verpackungssystem für die Aufbewahrung einer ophthalmischen Vorrichtung umfassend einen verschlossenen Behälter, welcher eine oder mehrere unbenutzte ophthalmische Vorrichtungen enthält, die in einer wässrigen Verpackungslösung umfassend ein Polymerisationsprodukt, welches aus einer Monomermischung enthalten wird, eingelegt sind, wobei die Monomermischung aus (a) einem zwitterionischen Monomer, das ein Zentrum permanenter positiver Ladung trägt, und (b) einem nicht-ionischen ethylenisch ungesättigten Monomer besteht, wobei die Lösung eine Osmolalität von wenigstens etwa 200 mOsm/kg, einen pH von etwa 4 bis etwa 9 aufweist und hitzesterilisiert ist,
wobei das nicht-ionische ethylenisch ungesättigt Monomer ausgewählt ist aus der Gruppe bestehend aus einem Hydroxyalkyl(alk)acrylat, einem Hydroxyalkylacrylamid, einem N-Vinyllactam, einem Polyhydroxyl(alk)acrylat, einem Polyhydroxylacrylamid und Mischungen davon.

2. Das Verpackungssystem gemäß Anspruch 1, wobei das zwitterionische Monomer eine quartäre Ammoniumeinheit und eine Phosphateinheit umfasst.

3. Das Verpackungssystem gemäß Anspruch 1, wobei das zwitterionische Monomer ausgewählt ist aus der Gruppe bestehend aus 2-Methacryloxyethylphosphorylcholin, 1-(4(4'-Vinylbenzyloxy)butan)-2'(trimethylammonium)ethylphosphate, [3-(Methacryloylamino)propyl]dimethyl(3-sulfopropyl)ammoniumhydroxid-Salz, Dimethylaminoethylmethacrylatdiethylsulfate und Mischungen davon.

4. Das Verpackungssystem gemäß Anspruch 1, wobei das Polymerisationsprodukt in der Lösung in einer Menge von etwa 0,005 Gew.-% bis etwa 1 Gew.-% anwesend ist.

5. Das Verpackungssystem gemäß Anspruch 1, wobei die Lösung weiterhin einen Puffer umfasst.

6. Das Verpackungssystem gemäß Anspruch 5, wobei der Puffer eine Borat- oder Phosphatverbindung umfasst.

7. Das Verpackungssystem gemäß Anspruch 1, wobei die Verpackung anschließend an das Verschließen der Verpackung hitzesterilisiert wird.

8. Das Verpackungssystem gemäß Anspruch 1, wobei die Lösung keine wirksame Menge eines Desinfektionsmittels enthält.

9. Das Verpackungssystem gemäß Anspruch 1, wobei die Lösung kein Germizid enthält.

10. Das Verpackungssystem gemäß Anspruch 1, wobei die ophthalmische Vorrichtung eine Kontaktlinse ist.

11. Das Verpackungssystem gemäß Anspruch 1, wobei die ophthalmische Vorrichtung eine anionische Kontaktlinse ist.

12. Ein Verfahren zur Behandlung einer biomedizinischen Vorrichtung, das Verfahren umfassend:
Kontaktieren einer biomedizinischen Vorrichtung mit einer Lösung umfassend ein Polymerisationsprodukt erhalten aus einer Monomermischung bestehend aus (a) einem zwitterionischen Monomer, das ein Zentrum permanenter positiver Ladung trägt, und (b) einem nicht-ionischen ethylenisch ungesättigten Monomer,
wobei das nicht-ionische ethylenisch ungesättigt Monomer ausgewählt ist aus der Gruppe bestehend aus einem Hydroxyalkyl(alk)acrylat, einem Hydroxyalkylacrylamid, einem N-Vinyllactam, einem Polyhydroxyl(alk)acrylat, einem Polyhydroxylacrylamid und Mischungen davon.

13. Das Verfahren gemäß Anspruch 12, umfassend:
Einlegen der biomedizinischen Vorrichtung in die Lösung,
Verpacken der Lösung, welche die biomedizinische Vorrichtung in einer Verpackungslösung enthält, auf eine Art und Weise, welche die Kontamination der Vorrichtung durch Mikroorganismen verhindert und
Sterilisieren der verpackten Lösung und Vorrichtung.

14. Das Verfahren gemäß Anspruch 12, umfassend:
Tränken der biomedizinischen Vorrichtung in der Lösung;
Entfernen der biomedizinischen Vorrichtung aus der Lösung und
Einlegen der biomedizinischen Vorrichtung direkt in das Auge.

15. Das Verfahren gemäß Anspruch 12, wobei die biomedizinische Vorrichtung eine Kontaktlinse ist.

16. Eine ophthalmisch verträgliche Mehrzwecklösung umfassend (a) ein Polymerisationsprodukt erhalten aus einer Monomermischung bestehend aus einem zwitterionischen Monomer, das ein Zentrum permanenter positiver Ladung trägt, und einem nicht-ionischen ethylenisch ungesättigten Monomer und (b) einen ophthalmisch verträglichen Träger für das Polymerisationsprodukt,
wobei das nicht-ionische ethylenisch ungesättigte Monomer ausgewählt ist aus der Gruppe bestehend aus einem Hydroxyalkyl(alk)acrylat, einem Hydroxyalkylacrylamid, einem N-Vinyllactam, einem Polyhydroxyl(alk)acrylat, einem Polyhydroxylacrylamid und Mischungen davon.

17. Die ophthalmisch verträgliche Mehrzwecklösung gemäß Anspruch 16, wobei das zwitterionische Monomer eine quartäre Ammoniumeinheit und eine Phosphateinheit umfasst.

18. Die ophthalmisch verträgliche Mehrzwecklösung gemäß Anspruch 16, wobei das zwitterionische Monomer ausgewählt ist aus der Gruppe bestehend aus 2-Methacryloxyethylphosphorylcholin, 1-(4(4'-Vinylbenzyloxy)butan)-2'(trimethylammonium)ethylphosphat, [3-(Methacryloylamino)propyl]dimethyl(3-sulfopropyl)ammoniumhydroxid-Salz, Dimethylaminoethylmethacrylatdiethylsulfat und Mischungen davon.

19. Die ophthalmisch verträgliche Mehrzwecklösung gemäß Anspruch 16, weiterhin umfassend einen oder mehrere antimikrobielle Wirkstoffe.

## Revendications

1. Système de conditionnement pour le stockage d'un dispositif ophtalmique comprenant un récipient scellé contenant un ou plusieurs dispositifs ophtalmiques non utilisés, immergés dans une solution de conditionnement aqueuse comprenant un produit de polymérisation obtenu à partir d'un mélange de monomères constitué (a) d'un monomère zwittérionique portant un centre de charge positive permanente et (b) d'un monomère non ionique à insaturation éthylénique, dans lequel la solution a une osmolalité d'au moins environ 200 mOsm/kg, un pH d'environ 4 à environ 9, et est stérilisée à la chaleur,
dans lequel le monomère non ionique à insaturation éthylénique est choisi dans le groupe constitué par un (alk)acrylate d'hydroxyalkyle, un hydroxyalkyl-acrylamide, un N-vinyl-lactame, un (alk)acrylate polyhydroxylé, un polyhydroxy-acrylamide, et leurs mélanges.

2. Système de conditionnement selon la revendication 1, dans lequel le monomère zwittérionique comprend un fragment ammonium quaternaire et un fragment phosphate.

3. Système de conditionnement selon la revendication 1, dans lequel le monomère zwittérionique est choisi dans le groupe constitué par la 2-méthacryloxyéthylphosphorylcholine, le phosphate de 1-(4-(4'-vinylbenzyloxy)butane)-2'-(triméthylammonium)éthyle, un sel d'hydroxyde de [3-(méthacryloylamino)propyl]diméthyl(3-sulfopropyl)ammonium, le diéthylsulfate de méthacrylate de diméthylaminoéthyle, et leurs mélanges.

4. Système de conditionnement selon la revendication 1, dans lequel le produit de polymérisation est présent dans la solution en une quantité d'environ 0,005 % p/p à environ 1 % p/p.

5. Système de conditionnement selon la revendication 1, dans lequel la solution comprend en outre un agent tampon.

6. Système de conditionnement selon la revendication 5, dans lequel l'agent tampon comprend un composé borate ou phosphate.

7. Système de conditionnement selon la revendication 1, dans lequel le conditionnement est stérilisé à la chaleur après scellage du conditionnement.

8. Système de conditionnement selon la revendication 1, dans lequel la solution ne contient pas une quantité désinfectante efficace d'un agent désinfectant.

9. Système de conditionnement selon la revendication 1, dans lequel la solution ne contient pas de composé germicide.

10. Système de conditionnement selon la revendication 1, dans lequel le dispositif ophtalmique est une lentille de contact.

11. Système de conditionnement selon la revendication 1, dans lequel le dispositif ophtalmique est une lentille de contact anionique.

12. Procédé pour traiter un dispositif biomédical, lequel procédé comprend la mise en contact d'un dispositif biomédical avec une solution comprenant un produit de polymérisation obtenu à partir d'un mélange de monomères constitué (a) d'un monomère zwittérionique portant un centre de charge positive permanente et (b) d'un monomère non ionique à insaturation éthylénique,
dans lequel le monomère non ionique à insaturation éthylénique est choisi dans le groupe constitué par un (alk)acrylate d'hydroxyalkyle, un hydroxyalkyl-acrylamide, un N-vinyl-lactame, un (alk)acrylate polyhydroxylé, un polyhydroxy-acrylamide, et leurs mélanges.

13. Procédé selon la revendication 12, comprenant :
l'immersion du dispositif biomédical dans la solution ;
le conditionnement de la solution contenant le dispositif biomédical dans une solution de conditionnement d'une manière empêchant une contamination du dispositif par des micro-organismes ; et
la stérilisation de la solution et du dispositif ainsi conditionnés.

14. Procédé selon la revendication 12, comprenant :
le trempage du dispositif biomédical dans la solution ;
le retrait du dispositif biomédical hors de la solution ; et
le placement du dispositif biomédical directement dans l'oeil.

15. Procédé selon la revendication 12, dans lequel le dispositif biomédical est une lentille de contact.

16. Solution multi-usages acceptable du point de vue ophtalmique, comprenant (a) un produit de polymérisation obtenu à partir d'un mélange de monomères constitué d'un monomère zwittérionique portant un centre de charge positive permanente et d'un monomère non ionique à insaturation éthylénique, et (b) un véhicule acceptable du point de vue ophtalmique pour le produit de polymérisation,
dans laquelle le monomère non ionique à insaturation éthylénique est choisi dans le groupe constitué par un (alk)acrylate d'hydroxyalkyle, un hydroxyalkyl-acrylamide, un N-vinyl-lactame, un (alk)acrylate polyhydroxylé, un polyhydroxy-acrylamide, et leurs mélanges.

17. Solution multi-usages acceptable du point de vue ophtalmique selon la revendication 16, dans laquelle le monomère zwittérionique comprend un fragment ammonium quaternaire et un fragment phosphate.

18. Solution multi-usages acceptable du point de vue ophtalmique selon la revendication 16, dans laquelle le monomère zwittérionique est choisi dans le groupe constitué par la 2-méthacryloxyéthylphosphorylcholine, le phosphate de 1-(4-(4'-vinylbenzyloxy)butane)-2'-(triméthylammonium)éthyle, un sel d'hydroxyde de [3-(méthacryloylamino)propyl]diméthyl(3-sulfopropyl)ammonium, le diéthylsulfate de méthacrylate de diméthylaminoéthyle, et leurs mélanges.

19. Solution multi-usages acceptable du point de vue ophtalmique selon la revendication 16, comprenant en outre un ou plusieurs agents antimicrobiens.
